(19) Europäisches Patentamt / European Patent Office / Office européen des brevets

(11) **EP 3 370 652 B1**

(12) **EUROPEAN PATENT SPECIFICATION**

(45) Date of publication and mention
of the grant of the patent:
**01.04.2020 Bulletin 2020/14**

(21) Application number: **16815900.2**

(22) Date of filing: **03.11.2016**

(51) Int Cl.:
*A61F 2/30* (2006.01)  *A61B 17/00* (2006.01)
*A61F 2/28* (2006.01)  *A61F 2/46* (2006.01)
*A61L 31/02* (2006.01)  *A61L 31/06* (2006.01)
*A61L 31/10* (2006.01)  *A61L 31/14* (2006.01)

(86) International application number:
**PCT/GB2016/053427**

(87) International publication number:
**WO 2017/077315 (11.05.2017 Gazette 2017/19)**

(54) **BONE IMPLANT**

KNOCHENIMPLANTAT

IMPLANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.11.2015 GB 201519451**

(43) Date of publication of application:
**12.09.2018 Bulletin 2018/37**

(73) Proprietor: **Attenborough Dental Laboratories
Limited
Nottingham NG1 3BN (GB)**

(72) Inventor: **ATTENBOROUGH, Edward John
Nottingham NG1 3BN (GB)**

(74) Representative: **Loven, Keith James
LOVEN Patents & Trademarks Limited
51 Wragby Road
Sudbrooke
Lincoln, Lincolnshire LN2 2QU (GB)**

(56) References cited:
WO-A1-2009/126820    US-A- 5 037 442
US-A1- 2004 053 196    US-A1- 2015 105 862

• ENDRES KIRA ET AL: "A new adhesive technique
for internal fixation in midfacial surgery",
BIOMEDICAL ENGINEERING ONLINE, BIOMED
CENTRAL LTD, LONDON, GB, vol. 7, no. 1, 19 May
2008 (2008-05-19), page 16, XP021036713, ISSN:
1475-925X

**Description**

**Field of the Invention**

The present invention relates to an implant for use in the body and a method of surgery using said implant.

**Background to the Invention**

In cases of injury, disease or trauma it is common for body parts to be supported in some form or other. Typical supports range from metal implants to venous tubes and the indications that require support range from broken legs, to tumour invasion damage to venous embolism. Supporting implants in most cases are physically retained in position, either by stitching or gluing. Orthopaedic implants are commonly fastened in place using screws or bolts of various different designs.

Fracture fixation is one of the most common procedures in orthopaedic surgery. Osteosynthesis by plating and fixation screws is commonly the favoured technique, especially when absolute stability of the fracture site is required (e.g., intra-articular fractures). However, using fixation screws for securing implants to bones is associated with several disadvantages. Drill holes made for insertion of the screws weaken the bone's cortex and may result in further fracture in regions of thin cortical bone (e.g. osteoporotic bones, comminuted fracture fragments). Anatomical structures that lie in close proximity to the deep cortex (e.g., nerves, vessels) may be injured while drilling the far cortex or when measuring pilot-hole depth for screw insertion. Damage to the articular cartilage by screw protrusion into the joint may occur and is becoming more frequent with the increased prevalence of fixed angled locking implants. The fixation power of a screw relies on the thickness and strength of the cortical bone and is significantly reduced in the presence of thin or damaged cortex (e.g., osteoporosis, comminuted fragments).

Clinically, hardware failure and mal-union leads to persistent pain at the fracture site, reduced strength across the joint, restricted range of motion and cosmetic deformity, all of which have a major impact on patient life quality and could expose the patient to further operations. Furthermore, actually positioning the implant in place and drilling into the bone requires invasive open surgery, which takes considerable time and greatly increases the potential for secondary bacterial infection.

The use of adhesives offers an exciting alternative to the problems described above. Adhesives used in medicine include fibrin, methacrylates, cyanoacrylates and/or dentin bonding agents. Adhesives have been used in medical practice for many years but have only limited usage in the body. For example they are not currently used in fracture fixation. This is because previous generations of adhesives have not had sufficient bond strength and have been unable to bridge the gaps between the irregular surface of a bone and the implant itself. Currently, the biggest disadvantage of adhesive bonding is the requirement that the bonded surfaces are close fitting. Furthermore, it is still not clear whether it is safe to use a large volume of adhesive in the body. High volumes of cyanoacrylate adhesive generate unfortunate tissue responses and the response of the body to high levels of dentin adhesive is still unknown.

Another further problem with the use of adhesives in surgery is the curing time to establish effective placement. For large scale operations exposing bones and deep internal body cavities, the use of adhesives to place an implant is often discounted, as the curing time required would mean the internal parts of the body are exposed for too long. This thereby makes the risk for secondary infection too high. For some areas of surgery, adhesive curing is possible, such as in the mouth as it does not matter how long the internal tissues are exposed. In fact in these areas UV curing may be used, as this speeds up the adhesive process. However UV can only be used because the teeth, gums and applied false teeth are relatively UV transparent. Therefore adhesive laid behind them (i.e. under the tooth) will still cure using UV applied from the exterior of the tooth. In cases where implants are used that are opaque (i.e. metal implants), then light curing is not suitable. Also, whilst light curing is particularly suitable for oral use because the light can be shone from behind the implant, in general surgery it is often that the top surface of the tissues that is exposed, and getting behind the material, whether it be bone, skin or other tissue (which are all light transparent to a degree) is not possible. Documents Endres Kira et al., 2008, Biomedical Engineering online, Vol 7, pp16, and US2004/0053196 disclose biomedical implants and new techniques for internal fixation.

There has now been devised an implant and method of surgery which overcome and/or substantially mitigate the above referenced disadvantages associated with the prior art.

**Summary of the Invention**

In a first aspect of the invention there is provided an implant for use in the body the implant being characterised in that, the shape of at least one portion of the surface of the implant matches the surface shape of at least one portion of at least one body part; and the at least one portion of the surface of the implant is coated with a layer of adhesive.

The implant according to the invention is advantageous primarily because it provides the means for supporting body parts. The implant can be made functionally strong and so even major structural parts of the body can be supported.

The implant may be a plate.

In the context of the present invention, mutually engageable means that the shape of the at least one portion of the surface matches the surface shape of the body part. That is to say, the profile of the at least one portion of the surface face is a negative image of the profile of the body part. Mutually engageable may also mean that the implant is conformant with a body part.

It is therefore a further advantage of the invention that the at least one portion of the surface is a close fit to the body part of interest. In use, when the implant is affixed to the body part of interest this greatly improves the bonding strength of the adhesive and allows for accurate and solid positioning of the implant.

The at least one portion of the surface may be coated in a layer of adhesive. This allows the implant to be bonded to a body part in use.

The adhesive may be any dental, orthopaedic or other adhesive. Preferably the adhesive is cured by external input of a first energy. In order to get the first energy through to the adhesive, the implant preferably comprises at least one conduit whereby said first energy may be introduced into the adhesive. The at least one conduit extends through the body of the implant and opens at the at least one portion of the surface. This has the benefit of reducing the curing time from normal conventional adhesive methods. Examples of suitable energy include but are not limited to electromagnetic radiation, ultrasound or thermal energies. The first energy which travels through the conduit diverges at it leaves the conduit and contacts the adhesive, causing it to cure rapidly. Furthermore, in forming a layer of adhesive a proportion of the energy is internally reflected at the adhesive boundary. This leads to further diffusion away from the conduit opening. In order to be cured by the input of a first energy the adhesive may comprise an initiator which is responsive to the energy. In one embodiment, the adhesive is cured by application of UV light from a UV light source. In such an embodiment the initiator is a photoinitiator which is activated by the UV light. Using UV light as the energy source the adhesive may cure within 90 seconds. In another embodiment, the adhesive is cured by ultrasound energy from an ultrasound source. Ultrasound energy has the benefit that energy is only provided to the places where there are boundaries between materials, such as the bone adhesive boundary or the implant adhesive boundary. Thus, ultrasound energy provides for a very targeted form of energy which reduces curing time and is less damaging to the surrounding internal tissues. The adhesive may be selected from the group consisting of acrylate or methacrylate adhesives, dentine bonding agents, including but not limited to glass-ionomer composites, and bone cements. Typical adhesive components include cyanoacrylate, methyl-methacrylate, octyl-cyanoacrylate, N-butyl-cyanoacrylate, dimethacrylate and pentaerythritol triacrylate.

Preferably the implant comprises a flange extending around the periphery of the implant and protruding in a direction approximately at right angles away from the implant. When in use the flange will therefore protrude towards the body and away from the at least one portion of the surface. The flange provides a stand-off between the at least one portion of the surface and the body part, when the implant is in use. This means that the user can apply maximum pressure to the rearward parts of the implant in order to affix the implant to the body part of interest so as to guarantee that the adhesive adheres fully and without risk of squeezing out all of the adhesive. The flange thereby controls the thickness of adhesive in use. Typically the flange extends between 50□m□and 200□m away from the surface of the implant. In use this therefore creates a gap for adhesive of the same dimensions. It will be recognised that even with the most modern methods of determining the surface shape of the body part of interest, there is some inherent error. Thus there is a possibility that even though the implant is mutually engageable, there will be some peaks and troughs in the profile of the body part which are not accounted for. This creates a non-uniform layer of adhesive which can lead to weakness. The flange allows for these errors and controls the thickness of the adhesive between the at least one portion of the surface and the body part at all times, even when maximum pressure is applied to the implant, in order to ensure that even the tallest peaks in the body part still have some adhesive over them when the implant is applied.

Preferably the implant comprises one or more protrusions positioned away from the periphery of the implant and protruding in a direction approximately at right angles away from the implant. When in use the protrusions will therefore protrude towards the body and away from the at least one portion of the surface. The protrusions provide increased structural integrity to the implant and preserve the integrity of the adhesive gap over substantially all of the surface of the implant. The protrusions also provide a point of contact between the body part and the implant itself, which in the case of bone implants, aids osseointegration.

The body part may be any part of the body. Examples of the body part include, but are not limited to, cartilage, soft tissue, ligaments, and tendons. Typically the body part is bone. The advantage of attachment to bone is that bone provides the most stable surface in the body to mount the implant on and provide the supporting function. For example, the body part may be a vertebra. The body part may be of any animal including human.

Preferably, the shape of the at least one portion of the surface is formed in accordance with a set of data derived from the body part in three dimensions. This avoids the need to make physical moulds or impressions to match the shape of the surface to. However the use of physical moulds or impressions is not discounted for the purposes of the invention. Also, deriving the surfaces from a set of data is much safer, more reproducible, cost effective and accurate than physical moulding.

Preferably the set of data is obtained by computer tomography (CT), ultrasound imaging, cone beam computed tomography (CBCT) and/or magnetic resonance imaging (MRI). The set of data may be obtained by any of infra-red imaging, optical or any other imaging modality or combination of modalities including but not limited to CBCT and optical merged together, including CT and ultrasound merged together, CT and MRI merged together.

Recent advances in imaging and manufacturing technologies make such customisation a reality. It is now possible to perform high resolution, thin slice, CT scans (slice thickness of 0.25 mm). The scans together with 3D imaging software can be used to reconstruct the anatomical shape of the requisite resolution prior to the disease, treatment, trauma or damage (i.e. fracture in the case of bone).

The implant may be manufactured by subtractive methods, including but not limited to milling, grinding, laser cutting, laser ablation and/or water jet cutting. This method makes the manufacturing of the implant quick, efficient, accurate and less expensive than prior art techniques. The implant may also be manufactured by additive manufacturing (examples include, but are not limited to, poly-jet printing, stereolithography, 3D printing, extrusion, sintering, selective laser melting, direct metal laser sintering, selective laser sintering, electron beam melting, fused deposition modelling and fused filament fabrication). The additive manufacturing technologies described above generally improve the economic feasibility of the manufacture of the implant. It is therefore now possible to produce metal implants as described herein with the same strength, accuracy (0.025 mm) and surface finishing (e.g. porosity) as machined (milled) ones, but at a fraction of the cost. The low production cost is gained as no set-up time is needed and there is negligible material loss in production as compared with milling. Also, with additive manufacturing the internal structure can be manufactured as trabecular structures or other structures which promote osseointegration without the need for etching, blasting or abrasive media. The implant may also be manufactured by swaging or pressing or forging or casting.

The implant may be made from any material which has sufficient structural integrity for the support role it might play when placed in the body. The implant may be made from metal. Preferably the implant is made from titanium, titanium alloy, PEEK and/or tissue/bone substitute. PEEK stands for Polyether Ether Ketone. The tissue/bone substitute may be natural or artificial.

The implant may be made from composite materials (examples include but are not limited to glass fibre, carbon fibre, or fibre reinforced plastics), thermoset polymer materials (including plastics), acrylics (examples include but are not limited to poly(methylmethacrylate) (PMMA), ceramics (examples include but are not limited to zirconium oxide, aluminium oxide, or lithium disilicate) or osseoconductive and osseointegrating materials (examples include but are not limited to artificial and natural bone augmentation materials). These materials are relatively inert when placed in the body, do not corrode and have the structural capacity required, for example when supporting bone or limb structures.

The at least one portion of the surface of the implant may be energy reflective. This means that the energy is internally reflected within the adhesive gap between the body part and the implant more efficiently and to a greater extent in use. For this to happen, the implant may comprise a reflective coating. This assists the adhesive curing process.

The implant may be opaque or impart a degree of transparency or may be completely transparent to energy. The transparency feature allows greater diffusion of the energy, but also allows for correct positioning of the implant against the body part. For example, the implant may be made from radio-opaque materials so that they are visible with X-ray scanning post-surgery. Alternatively they may be made radio-translucent but coated in a layer of a radio opaque substance e.g.: BaSO4 or radio-opaque markers, so that the position of the implants can be tracked post placement by scanning techniques

It will be recognised that the at least one portion of the surface of the implant could have a relatively large surface area compared to the whole surface area of the implant as a whole and the part of the body. Therefore there may be more than one conduit in the implant, to allow energy through to the adhesive in more than one place over the surface of the implant. This has the effect of reducing the curing time. The one or more conduits are preferably equal to or less than 2mm in diameter. This diameter is sufficient to allow light in but not too large to cause structural or bonding failure. The diameter of the conduits is however dependent on the thickness of the implant and what the implant is to be used for. Therefore for the purposes of the invention the diameter of the conduits may be greater than 2 mm.

When there is more than one conduit each conduit is preferably separated from its nearest neighbour by at least 5 mm. For example each conduit may be separated from its nearest neighbour by between 10 and 100mm, inclusive. This has the benefit that the energy is able to fully saturate the adhesive without affecting the structural integrity of the implant in any way. The separation distance of each conduit is not fixed though, and depending on the circumstances each conduit may be separated from its nearest neighbour by less than 5 mm. It is recognised also that the adhesive may only be coated on specific portions of the implant around conduits which are separated by distances greater than 10 mm for example. Thus, whilst the implant may fit over the entire area of a body part, it may be only adhered at specific places on the body part.

The implant may be used in vivo or ex vivo. Although the implant may be used in a wide variety of applications, the areas in which it is currently envisaged that it will be most valuable are on correcting pars fractures, lamina defects, vertebral abnormalities, correcting deformities, fracture reduction, cosmetic and/or aesthetic improvement and/or improvement in function (e.g. joints). Other examples of where the implant may be used include, but are not limited to, cartilage (rhinoplasty, joint surface replacement), all orthopaedic parts of the body, custom maxillo facial applications and custom made patient specific flexible scaffolds for soft tissue repair (e.g. hernia, skin damage). The implant may comprise attachment parts for coupling of the implant to other components. The other components includes superstructures. Example of superstructures include but are not limited to bone anchors, artificial joints, or bone augmentation devices to strengthen weak but un-fractured bone or correct asymmetry or deformity. The attachment parts may be selected from the group consisting of an eyelet connection, a rod connection, an abutment, an eyelet, a loop, a hole and bushing, a ball connection and a drilling, tapping and/or cutting guide.

In another aspect not being part of the invention there is provided a surgical method comprising the steps of:

a) forming an implant comprising at least one portion of the surface of the implant being mutually engageable with at least one portion of at least one body part,
b) applying a layer of adhesive to the at least one portion of the surface,
c) engaging the at least one portion of the surface with the at least one portion of at least one body part.

The method may be a method of treatment. The method is advantageous primarily because it eliminates the need for screws or other physical attachments which would otherwise further weaken the existing bone. Also, the mutually engageable nature of the implant spreads the fixation forces over the entire contact area. The load on the body part is therefore reduced and this minimises the risk for hardware failure.

Prior to the application of adhesive to the implant, a bonding agent may be applied to both the surface of the body part and the surface of the implant. The bonding agent preferably comprises a surface preparation material which cleans the surface of the body part and the implant. This leads to greater and more efficient adhesion of the implant to the body part. The bonding agent preferably comprises hydroxyethyl methacrylate, methacryloyloxydecyl dihdrogen phosphate, hydrophilic aliphatic dimethacrylate and/or dl-camphorquinone.

Also prior to engaging the implant with the body part, the at least one portion of at least one body part is coated with a layer of adhesive. This increases strength of the bond.

In the method the implant is preferably formed substantially as described above. This has the benefit that it combines custom implants and adhesive bonding to eliminate the most serious complications of fixing and supporting body parts, such as fractured or osteoporotic bone. It also enables implants to be fixed in vivo and obtain the required adherence after exposing the implant to external energy for a much shorter time than is conventionally used and where full cure can take up to 24 hours. This therefore greatly reduces the time the individual has in surgery.

In the method the adhesive may be curable by external input of energy and the implant comprise at least one conduit extending through the body of the implant and opening at the at least one portion of the surface. In such a scenario the method comprises the further step of exposing the adhesive in the region between the at least one portion of at least one body part and the at least one portion of the surface to external energy from an external energy source presented to the at least one conduit from the exterior of the implant.

In the method as described above the first external energy is substantially as described above.

Preferably the adhesive is exposed to a first external energy by inserting one end of an energy conducting medium, or rod or tube into the conduit. Where the external energy is UV light, preferably the adhesive is exposed to UV by inserting one end of a UV conducting rod into the conduit. The opposite end of said rod or tube is connected to an energy source (such as a UV light source in the case of UV energy). The rod or tube may also be used to position the implant against the body part in use. Thus the rod or tube serves the purpose of an applicator. To achieve all this, the rod or tube may be releasably engaged with the conduit. For example the rod or tube may comprise a male end, which engages with a mating female part in the conduit. Said male and female parts are preferably lockable and unlockable. This may be achieved by a twist lock feature between the male and female parts.

The end of the rod or tube which is disposed within the conduit may comprise a detector to detect the amount of energy delivered through the conduit to the adhesive. The detector may also detect the amount of energy absorbed by the adhesive. The detector may also detect energy it receives from neighbouring conduits. This provides an accurate way of ensuring the adhesive has fully cured. In the case of UV, optical or IR energy, the detector may be a photocell present on the end of the light rod.

The adhesive may be deactivated by exposure to a second external energy, wherein the first and second energies are different. The second energy may be chosen from the list of energies described above. The adhesive may comprise a deactivation agent, such as a denaturing agent with is responsive to the second energy. Deactivated in the context of the present invention means to lose power of adhesion after curing. This has the benefit that the implant can be removed from the body part of interest without causing any secondary trauma when the implant is required to be removed. Thus there is provided a method of removing an implant adhered to a body part by adhesive, wherein removal of said implant is affected by the application of a second energy to the cured adhesive. The implant may be substantially as described above and may be used in the method also substantially as described above.

The second energy may also be delivered to the adhesive through the conduit using a similar rod or tube as is described above for the application of the first energy. This makes it easier to connect an energy source to the implant. In one example, the second energy is radiated infrared light which is delivered down a light pipe through the conduits in the implant. The infrared light heats up the adhesive causing it to denature and release from the body part. In another example, the second energy is ultrasound energy delivered to the adhesive through the implant using a transducer, horn and interface media, e.g. oil/petroleum jelly. The second energy may be delivered to the adhesive by contacting the implant with a heated thermal element. As the thermal element is heated the energy is conducted into the implant and spreads to the adhesive over the surface causing it to deactivate. Where the second energy is thermal energy (either from ultrasound or otherwise), the heat ranges used are more than body temperature but conveniently less than that which would cause damage or necrosis of the body part in use through brief exposure. Preferably the implant and/or the adhesive are heated to between 50 - 70 °C inclusive to effect removal of the implant.

The adhesive may also be deactivated chemically. Such chemicals include releasing agents, denaturing agents and etching materials.

The implant may be removed after deactivation of the adhesive using a rod or tube substantially as described above.

The present invention therefore provides a method of removal of an implant.

The implant according to the invention may be retained in place solely by the adhesive. The implant according to the invention may be retained in place by a combination of the adhesive and at least one screw. The combination of adhesive and screws means that the screw depth and number can be reduced whilst producing the same or greater fixation forces.

In another aspect not being part of the invention there is provided a software model to design the implant substantially as described above. The software can be used to generate the specifications for the implant given its eventual purpose and position in the body. In particular the software may calculate the optimum distribution and positions for the one or more conduits, in order that the first and/or second energy is introduced into the adhesive as effectively as possible. The software may also calculate and specify the optimum dimensions of the conduits and/or the thickness of the peripheral flange. The software model may also validate the correct placement of the implant in-silico prior to surgical placement. The validation in this context means to model the correct placement of the implant to make sure that it will fit onto the body part when required to do so. The software model may also validate the structural integrity of the implant given the eventual purpose of the implant. Thus, data may be fed into the software model in terms of the predicted forces the implant may be subjected to and the model will be able to predict the required structural dimensions.

The software model may be personalised to patient specific scan data to provide a method of in-silico validation of the design and function of the implant. This includes number, size, separation and placement of the conduits to deliver optimal energy and full curing. It also provides validation of the implant fixture surface design to maximise adhesion, whilst delivering the requisite structural integrity and mechanical performance of the implant for the intended purpose. The software model uses finite element analysis (FEA), iterative solving and mathematical techniques to model the static and dynamic stresses and forces in the tissue, body part, the adhesive, the implant and the interface between each. In another embodiment the model may be personalised to patient specific scan and run with patient kinematics' / gait analysis data to predict susceptibility to patient body part failure which may be used to prescribe implants to avoid the risk of failure. One application of such an embodiment would be the avoidance or mitigation of potential stress fractures in human or animal competitive sports players.

The software model has a human-computer CAD interface employing parametric design. The model has parametric landmarks or handles for specific anatomy or anatomical features. When personalised with patient specific scan data, the model enables the measurement of the difference in position of these anatomical landmarks between the model and the actual patient anatomy. This is then used for automatic proposition of the implant design based on parametric design principles and FEA analysis, which greatly simplifies the design process.

The above validation of custom made patient specific implants in-silico may avoid and/or mitigate the need for animal testing.

### Description of the Drawings

The invention will now be described by way of example and/or illustration only with reference to the accompanying drawings in which:

Figure 1 shows an embodiment of the implant according to the invention adhered to a vertebra;

Figure 2 shows the results of experimental tests to demonstrate the tensile strength of the bonding achieved with and without the application of UV light to cure the adhesive;

Figure 3 shows an embodiment of the implant according to the invention adhered to the orbital floor;

Figure 4 shows an embodiment of the implant according to the invention adhered to the Malar region;

Figure 5 shows an embodiment of the implant according to the invention in the form of a TMJ replacement for a canine;

Figure 6 shows an embodiment of the implant according to the invention in the form of an elbow replacement;

Figure 7 shows an embodiment of the implant according to the invention used as an S spine support; and

Figure 8 shows an embodiment of the implant according to the invention in three dimensional view showing the flange.

Figure 9 shows a flow chart for the application of the software model according to the invention in optimising the design of the implant.

Figure 10 shows lateral, posterior and transparent views of an L4-S1 model.

Figure 11 is a table showing the material properties of different spinal components.

Figure 12 is a graph showing typical FE result for axial rotation.

Figure 13 is a table showing the comparison between experimental results, literature results and FE modelling results.

Figure 14 shows plots of von Mises stresses for typical loading conditions.

Figure 15 is a table showing typical fatigue parameters to predict the fatigue strength of bone.

Figure 16 shows plots of von Mises stresses in the high stress regions in the healthy lumbar human spine.

Figure 17 shows plots of shear stress in the high stress region in the healthy lumbar spine.

Figure 18 is a table showing the number of fatigue cycle for each loading condition of healthy lumbar spine.

Figure 19 shows a model of a laminar defect after "cut extrude".

Figure 20 shows plots of von Mises stresses shown in the high stress region in the lumbar spine having a laminar defect.

Figure 21 shows plots of shear stress in the high stress region in the lumbar spine with a laminar defect.

Figure 22 is a table which shows the number of fatigue cycle for each loading condition of lumbar spine with a laminar defect.

Figure 23 shows a flow chart describing the automatic design and learning program work flow.

## Detailed Description of the Illustrated Embodiment

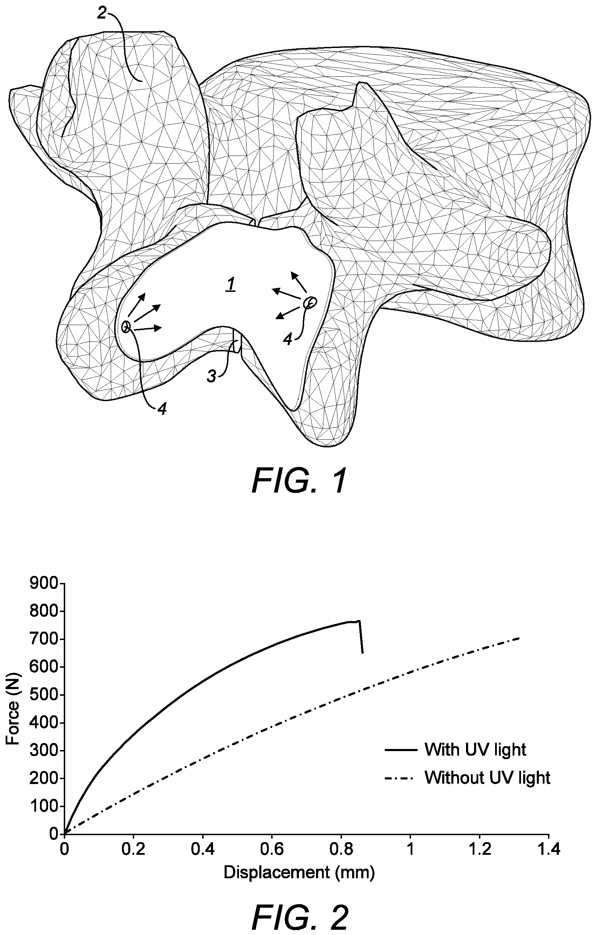

Figure 1 shows an embodiment of the implant according to the invention. The implant is generally designated 1. The implant is shown attached to a human vertebra 2 which has a lamina defect 3 caused by a stress fracture in the bone. The defect in this case is malformation of the two halves of the vertebra so that there is no structural connection present. This can be a source of lower back pain. However having one of these conditions does not mean that back problems are a certainty. It does however present a higher risk compared to people that don't have the defect, towards developing pain. These conditions can cause mechanical pain, which is the kind of pain that come from within the moving parts of the spine. These conditions can also cause compressive pain, which is derived from pressure on the nerves in the lower back.

The implant 1 is custom made, which means that it is manufactured to specifically fit against the individual vertebra shown with the inside surface of the implant matching the surface of the bone. The implant has an approximately uniform thickness of between 2 and 3 mm inclusive. The greater the fracture area the greater the thickness of the implant.

The implant is made from titanium alloy using a milling manufacturing process.

The implant 1 has two conduits 4 which extend through the whole thickness of the implant from the outward side (side shown) to the inward side (side in contact with the vertebra). Each conduit 4 is 2mm in diameter, and in the example shown they are positioned either side of the defect. This is because placing a conduit over the defect would reduce the strength of the supporting implant in the region where is required the most.

Between the implant 1 and the vertebra is a layer of medical grade adhesive which is applied to the implant 1 before the implant is applied to the vertebra. The adhesive is a UV curable adhesive.

All around the edge of the implant 1 there is flange 30, which may also be described as a protruding lip, which protrudes inwards towards the vertebra. For the sake of clarity, inwards in the context of the present invention means towards the body part in use.

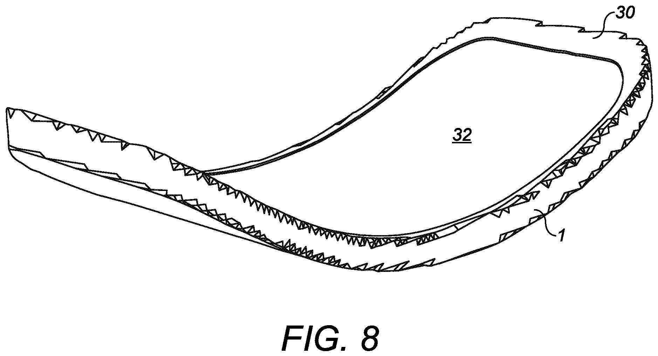

In use, prior to the application of adhesive the surface of the implant is coated in a layer of bonding agent. The adhesive is then applied to the under surface 32 of the implant 1. See figure 8 for detail of the under surface 32 and the flange 30. This is done using a conventional dispenser or by hand mixing the adhesive for 20 seconds and then pasting on with a rigid applicator. Prior to applying the implant in place against the bone the bone surface is prepared firstly by brushing the surface of the bone to remove the thin layers of soft tissue surrounding the bone. This reveals the native bone surface and roughens it microscopically to improve bond strength. Then the bone is coated in a layer of bonding agent. As the agent is liquid, a 3d printed silicone barrier dam is created and applied around the bonding area to prevent the liquid from damaging the surrounding tissues. The dam may be manufactured in substantially the same way as the implant is manufactured.

The implant 1 is then pressed into place against the vertebra 2. Maximum force can be applied to the implant 1 to ensure maximum adherence without risk of squeezing out all the adhesive because the flange around the edge contacts the vertebra and controls the separation of the vertebra with the under surface of the implant.

The inventors have also found a surprising effect of the combination of the at least one conduit and this flange. Effectively it means that when the user applies the implant to the body part, and applies pressure, the only place for the adhesive to escape is out of the conduit. Surprisingly, this actually increases the bond strength of the adhesive. It is thought that this is because of the increased surface areas generated by the conduits in combination with a vacuum effect.

A UV light pipe attached to a UV source is then engaged with each of the conduits 4 and UV light is delivered to each of the conduits simultaneously. The light pipe is a light conducting rod that delivers UV light to the UV activated adhesive. The adhesive is UV transparent and is able to transmit the UV through it. Furthermore, the inside surface of the implant is reflective. The combination of these features means that the UV light diffuses outwards from the conduit into the adhesive gap between the implant and the bone. It takes approximately 90 seconds for the adhesive to completely cure across the whole surface of the underside of the implant. The paths of light diffusion are represented by the arrows shown on the figure. The implant 1 is thereby set in place in contact with the vertebra and provides a supporting function within 90 seconds. This greatly reduces the time the patient spends in theatre. Using conventional methods it can take 24 hours for the adhesive to fully cure.

In order to manufacture the implant 1 so that the face of the implant is mutually engageable with the bone, firstly the body part is scanned. This can be done before surgery. Most modern methods of scanning will be suitable. In this case ultrasound, CT and/or MRI scanning are used. The scans produce a series of slices through the bone. The slices are combined to create a three dimensional image of the bone in detail. A three dimensional model of the implant is then created having a bone abutment surface that is a negative of the above three dimensional image of the bone surface. The model, which is present as a data file, is processed by a milling machine in order to then manufacture the implant. It will be appreciated that an additive process may also be used such as selected laser sintering for example. In the milling process a 3 or 5 axis miller progressively grinds away material from a block of material (in the same way that a sculptor will cut from a block of stone to create a bust) in accordance with the model to reveal the implant. Whichever process is used, the general process is the same, which involves the collection of medical data, the definition of the layers for the creation of the face of the implant, determining the optimal positions for the conduits and any other parts attached to the implant, determination of the depth of the flange and eventual manufacture.

It is recognised that due to the inaccuracy of even most modern scanning techniques the implant may not be an exact fit over the body part of interest. It may be, for example that there are peaks and troughs in the body part of interest, which are not replicated in the implant. Without the flange 30 around the rim of the implant, then it is likely that when the implant is applied to the body part of interest the implant would contact the bone at various points. In such places, the layer of adhesive is thinner and therefore weaker. The flange 30 of $80\mu$m, guarantees that the face of the implant always stood off from even the highest unaccounted for peaks in the bone's surface. Thus the amount of adhesive between the implant and bone is controlled and therefore the mechanical forces involved after curing are controlled too. Furthermore, as there are no bone peaks that contact the implant, there is an unhindered pathway for the UV light to travel through during curing. This means that UV light travels further in the adhesive gap and the adhesive can be fully cured within 90 seconds.

Using the method described above, the inventors completed a number of experimental tests to demonstrate the tensile strength of the bonding achieved. An implant was manufactured according to the method described above and bonded to a section of bone. UV light curing was used to cure the adhesive according to the method described above for 90 seconds. Successive retractive forces were applied and the displacement of the implant from the bone measured. The results are shown in Figure 2. The results show that the adhesive can resist a force of up to 750N with a displacement of only approximately 0.8 mm, before it breaks and the implant separates from the bone. A comparative test without the use of UV curing exhibited a substantially greater displacement when a force in excess of 500N is applied.

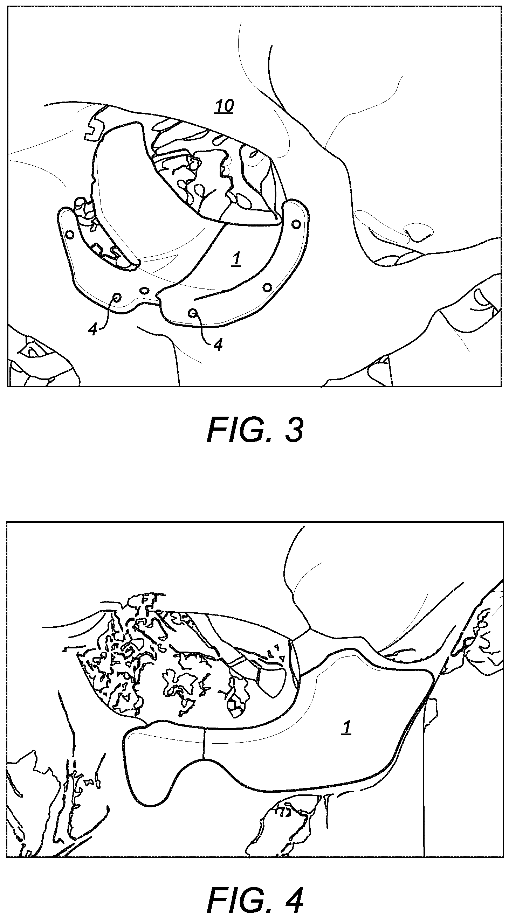

Figure 3 shows an embodiment of the implant 1 according to the invention adhered to the orbital floor in the skull 10.

Figure 4 shows an embodiment of the implant 1 according to the invention adhered to the malar region of the skull 10.

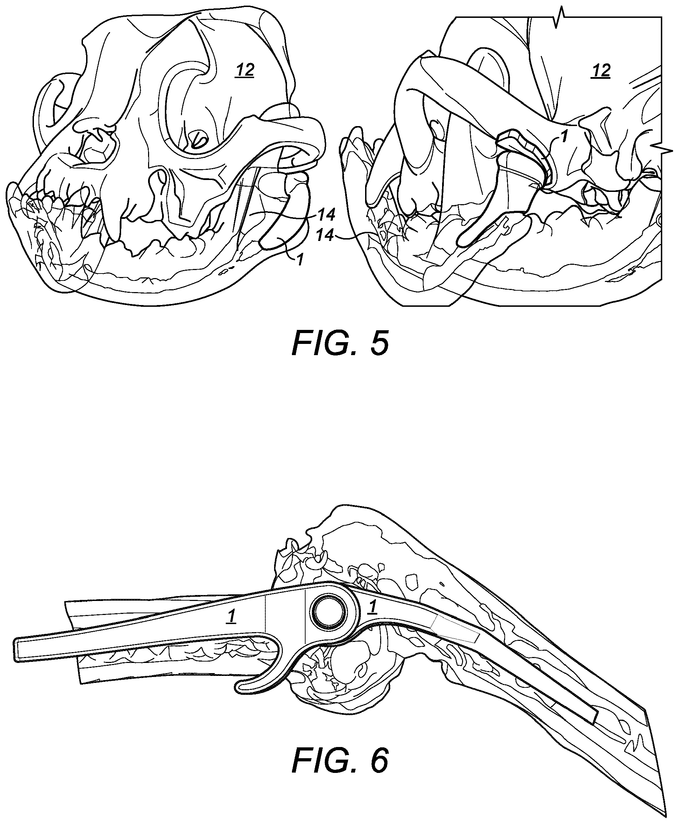

Figure 5 shows an embodiment of the implant 1 according to the invention in the form of a temporomandibular joint (TMJ) replacement for a canine. The left figure shows the view from the rear and the right figure shows the view from the front. The skull 12 and mandible parts 14 are shown joined by the new TMJ joint.

Figure 6 shows an embodiment of the implant according to the invention in the form of an elbow replacement. In this example the implant has been fixed into the central part of the existing bones and is in two halves which are joined by a hinge joint.

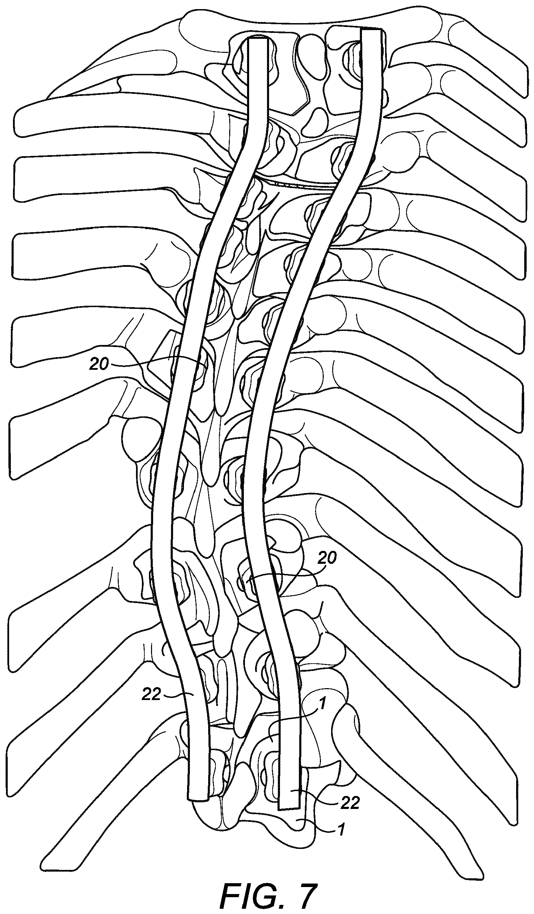

Figure 7 shows an embodiment of the implant 1 according to the invention used to fix a spine support. As is exemplified in this figure, the implants 1 have super structures 20 fixed to them. These super structures are linked by a brace 22. The brace 22 can have tension applied to it to straighten the spine, or can be used to stabilise damaged joints.

Figure 8 shows an embodiment of the implant 1 according to the invention in three dimensional view showing the flange 30. The flange 30 extends around the whole periphery of the implant and is 80μm deep. In use the adhesive is spread on the under surface 32 of the implant 1. The implant is applied to the body part with the under surface 32 downward. An adhesive gap is thereby created as the flange 30 is in contact with the body part around the edge of the implant, but the under surface 32 is held away from the body part. Only the under surface 32 is mutually engageable with the body part.

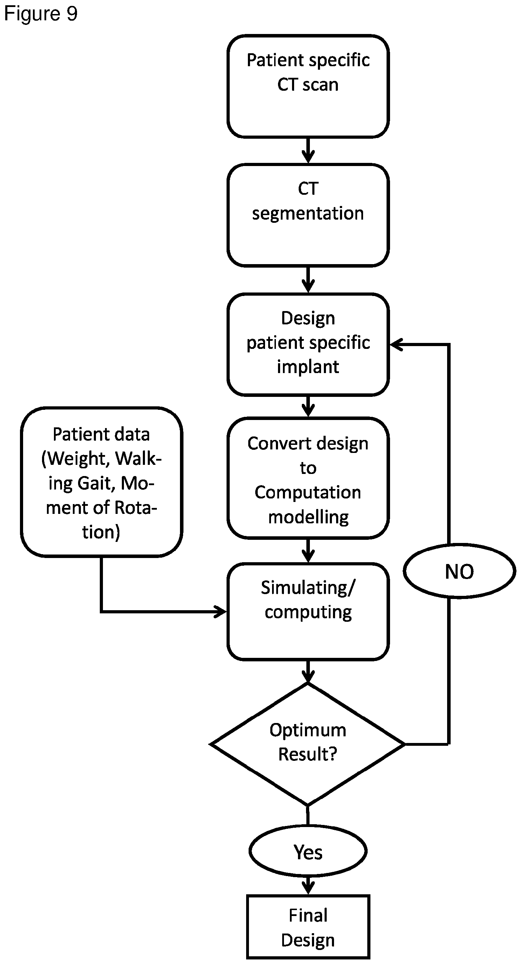

Figure 9 shows a flow chart for the application of the software model according to the invention in optimising the design of the implant. The process for optimising the design of the implant begins with a patent specific CT scan. This scan is then segmented and a patient specific implant is designed, substantially as described above. However, in order to optimise the design, the design is converted into a computer model which allows the application of simulation programs and computing procedures to produce the optimum result. If the optimum result is not achieved initially then further modelling is carried out. Inputs into the model include but are not limited to weight of the patient, walking gait data and moment of rotation. After successive rounds of optimisation a final design is produced, which is implemented into the construction of the implant.

Example of the software model and its use in response analysis of the lumbar spine during regular training of an athlete.

This example involved creating a finite element (FE) model of the lumbar spine including cortical bone, spongial bone, annulus, nucleus, and facet joint. The regions L4-S1 human lumbar spine were studied and the FE model simulated the loading pattern of a sport athlete. The patient data inputted included parameters of extra extension, flexion and axial rotation. Stress analysis of the lumbar spine was studied and used to predict further fatigue fracture risk in healthy individuals.

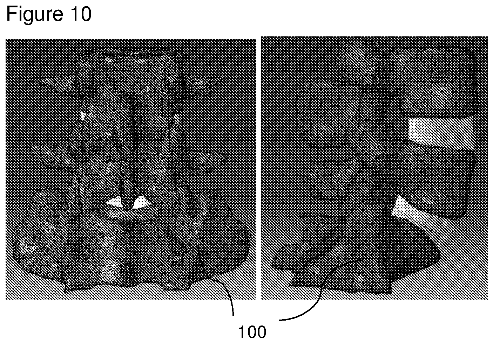

Figure 10 shows a three dimensional aspect of a nonlinear finite element model of a L4-S1 human lumbar motion segment 100 which was developed using finite element code ABAQUS 6.13. The lumbar spine model was modelled with solid tetrahedral linear elements (types C3D4). There are total 205422 elements in the model. The S1 is the final vertebrae which acts as a rigid body in motion with little deformation. The sacral slope was oriented to 66° mimicking the degree of sacral slope in athletes. A displacement of constraint 'Encaster' was used to define inferior vertebral of S1 to prevent any movement when loading is applied. Different loading conditions were used to describe the body movement including compressive force and torsional moment. The compressive force is representing the body weight of the upper body following by the torsional moment loading such as flexion, extension or axial rotation.

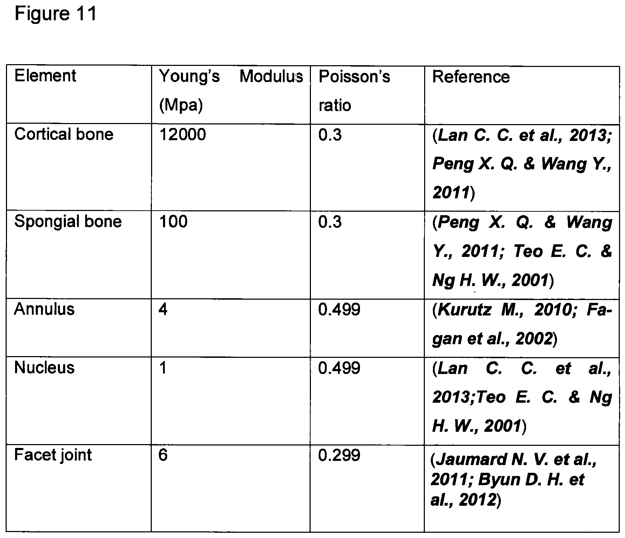

Different ranges of compressive forces and torsional moments were used to simulate a realistic load scheme of body movement. In this work, 800N of compressive force followed by 10Nm of torsional moment was applied to act as a 'safety factor' and take into account unexpected variation. Another reason for these amounts were to simulate a maximum loading value which is a prediction of the further failure risk of the bone. Through the ABAQUS load module, two types of load were created (concentrated force and Moment) and applied to the superior vertebral endplate of L4. As shown in Figure 10(b), red colour meshes represent cortical bone and white is spongial bone. The intervertebral regions were modelled with annulus (white) and nucleus (Green). The material properties had been assigned individually in the program and then assembled together according to the original coordinates or position of the human body. The annulus and nucleus was modelled as a low modulus nearly incompressible body with Poisson's ratio of 0.499. Figure 11 shows the material properties of each component.

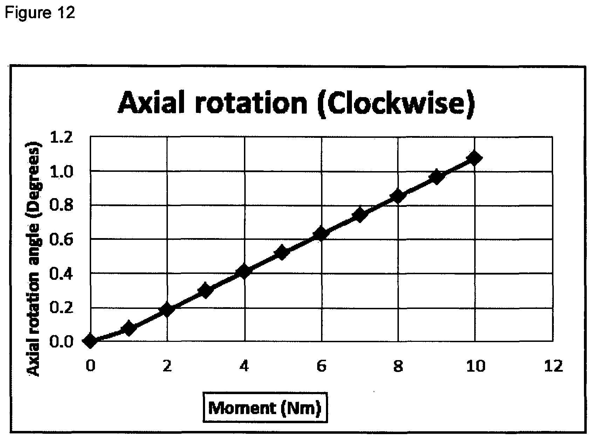

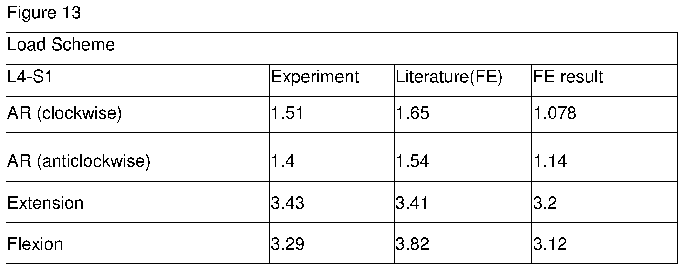

In this example, all the elements were assumed to be fully integrated. The surfaces were simulated by imposing a rough friction behaviour to the tiecontact pair to prevent extraction from each other. Figure 12 shows typical FE results for axial rotational angle of spine lumbar over torsional moment. Four loading types have been simulated here as shown in Figure 13. The table in figure 13 shows the comparison between literature and FE model results, which were very close to each other. The literature loading angle of torsional moment loading was used as a guide to validate the model.

Qualitative investigation of the stress features was used to predict the fracture according to the bone strength. von Mises stresses were used to study the load transfer and stress concentration. The general form of the von Mises stress is

$$\sigma v2 = 1\ 2[(\sigma 11 - \sigma 22)2 + (\sigma 22 - \sigma 23)2 + (\sigma 11 - \sigma 33)2 + 6(\sigma 232 + \sigma 312 + \sigma 122)]$$

$$(1)\ \text{(Eq. 1)}$$

Where $\sigma 11$, $\sigma 22$ and $\sigma 33$ is represent x,y and z principle stress, $\sigma 23$, $\sigma 31$ and $\sigma 12$ is represent yz,zx and xy shear stress.

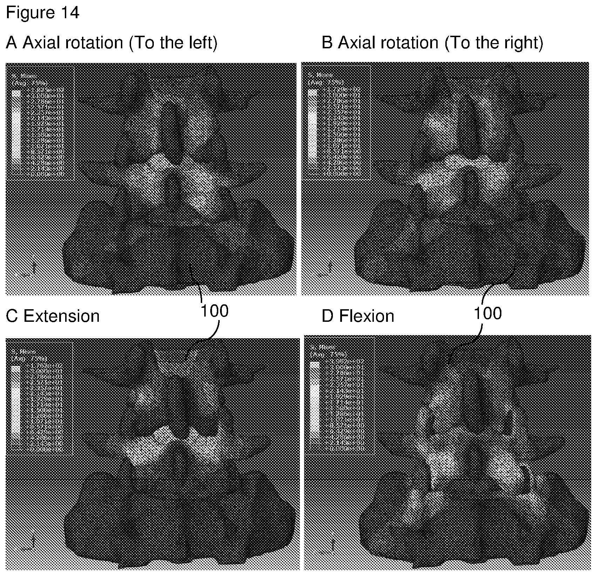

Looking at the same lumbar portion (L4-S1) 100, as shown in Figure 14, different colour contours represent the von Mises stress level starting from minimum in blue to maximum in red, as compressive load and torsional moment were applied to the L4 superior endplate. External compression and shear loading were transmitted from L4 superior endplate to L5 and L5 to S1 through the resistance of the intervertebral discs and the facet joint. Due to the fact that S1 was fixed and L4 is transmitting all the loading toward L5, L5 is sustaining all the loading transmitted from L4 and restricted movement by S1 superior articular process. Such a loading condition generates twisting moment of the pedicles region. As shown in Figure 14(a), an axial rotation (to the left) has been simulated and result shows that a high stress field is located by the right side of the laminar region of L5. Similar results were obtained for axial rotation (to the right) as shown in Figure 14(b). High stress occurred by the left par region of L5. Figure 14(c) is the extension loading scheme and the stresses are equal under compression on the par region of L5. For the flexion motion see Figure 14(d) which shows that the laminar region of L5 is under tension by the laminar region of L5. The results are sensible and show every single motion and different stress distributions on different regions of vertebrae.

Stress distributions are important to investigate and understand as the clinical consequence of excessive fatigue damage in bone. The stress fracture of the bone can occur when the material is continuously loaded to respective yield point. The higher the stress level, the more the bone behaves like a brittle material. The fatigue strength for compression is about 150Mpa. The fatigue behaviour of bone has been investigated in the form of Wohler curves (S-N curves) as represented by Equation 2 below.

$$S = S0 + Sr\log\ (Nf)\ \text{(Eq.2)}$$

$S0$=149.8 Mpa and $Sr$=-17.96 Mpa.

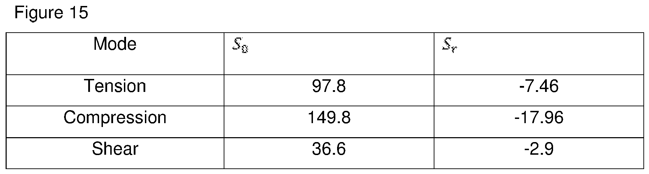

where S is the fatigue strength, Nf is number of cycles to failure, 50 is maximum fatigue strength and $Sr$ is slope of the fatigue curve. Figure 15 shows a table of typical fatigue parameters to predict the fatigue strength of bone and is one of the sample group taken from donors with the age of 53 and provides some experimental results for fatigue strength of the bone. Equation 2 predicts the fatigue strength of the simulation results. The 50 is a maximum strength in a single cycle failure where if the stress over or equal to the 50 then fail immediate within one cycle. Figure 14 is a plot of von Mises stress with colour contour to show the difference between stress levels of the spine lumbar by the laminar region.

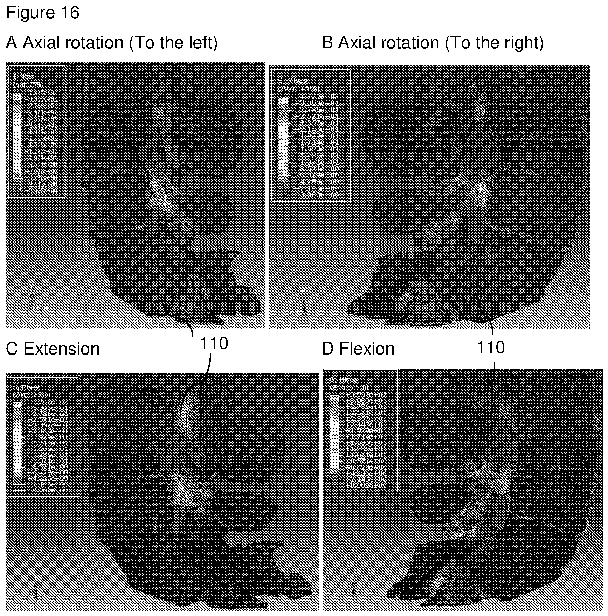

Figure 16 shows similar plots of von Mises stresses for the inner vertebral arch 110 of the lumbar spine. The label on the left of each of the figures shows the values of stress and what each colour correspond to. Figure 14(a), shows a typical loading type (Axial rotation (To the left)) with higher von Mises stress by the right side of the laminar (19.29Mpa) and inner pedicle of L5. For the axial rotation (To the right), stress is concentrated within left side of L5 between laminar and par region and is about 17.14Mpa as shown in Figure 14(b). From the inner view (Figure 16(b)) stress is occurred by the bottom left side of pedicle region of L5 (close to 30Mpa). Extension of the spine may cause compression on the L5 and Figure 14(c) shows the laminar region of L5 in both sides with the maximum stress of 19.29Mpa. Similar phenomena and stress (19.29Mpa) is shown for flexion loading (Figure 14(d)), but this time it is in tension stress due to force and moment bending downward and stop by the superior articular facet of S1 to form pulling effect. For Figure 16(c &d) the maximum stress is close to 30Mpa and occurred by the inner pedicle. This suggest that the inner pedicle region is major stress concentration region and not the laminar region.

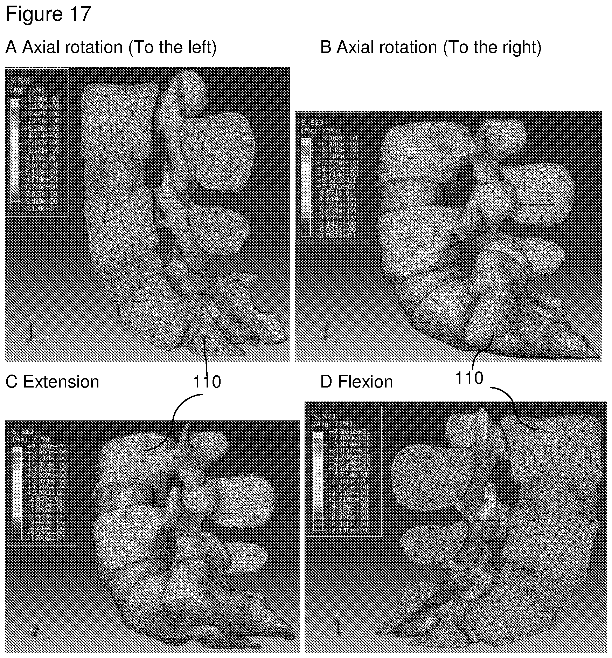

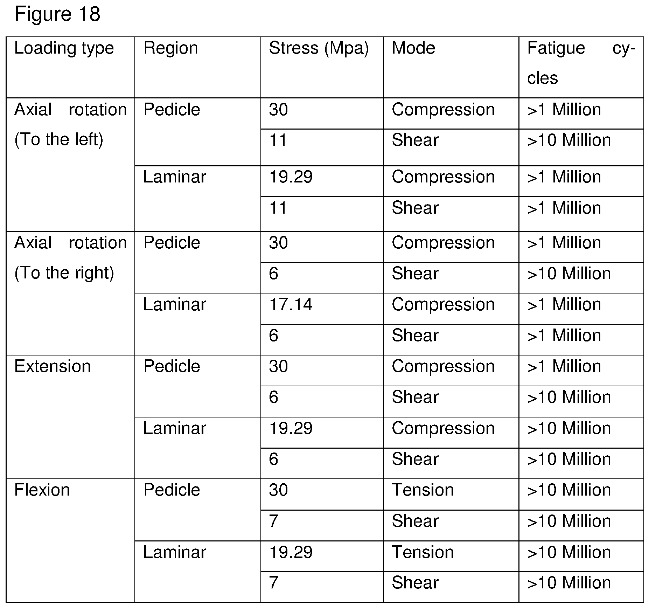

Figure 17(a-d) shows the shear stress of every loading condition at the inner vertebral arch 110 and the highest shear stress is about 11Mpa for axial rotation (To the left), as shown in Figure 17(a). Figure 18, is a table which shows the number of fatigue cycles for each loading condition of healthy lumbar spine based on Eq. 2. Although no single cycle failures were observed in any loading conditions, stresses that correspond to compression stress failure after 1 million cycles of repetitive axial rotation conditions case were observed. For extension and flexion loading conditions, fatigue failure only occur over 10 million cycles.

Example of the software model and its use in response analysis of the lumbar spine which has been modelled with laminar defects.

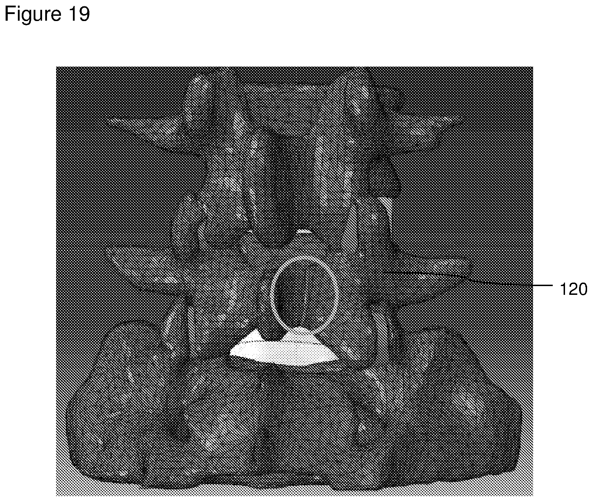

In this example, a 3D model of the lumbar spine with a laminar defect of L5 was developed and investigated for the influence of the defects in a substantially similar way as described above. A laminar defect was created through ABAQUS cut-extruded to dictate the area of fracture. To understand the stress distribution in L5 with one side laminar defect, FE modelling was used to mimic the laminar fracture by 'Cut Extrude' (blue circle) on right side of the L5 laminar region 120 as shown in Figure 19. The purpose of this study was to find out the further risk of fracture in the laminar defect on right side of L5 vertebrae. The mesh C3D4 tetrahedron element type was used in this model. Similar material properties and interaction properties were used in this model to assess the comparability results.

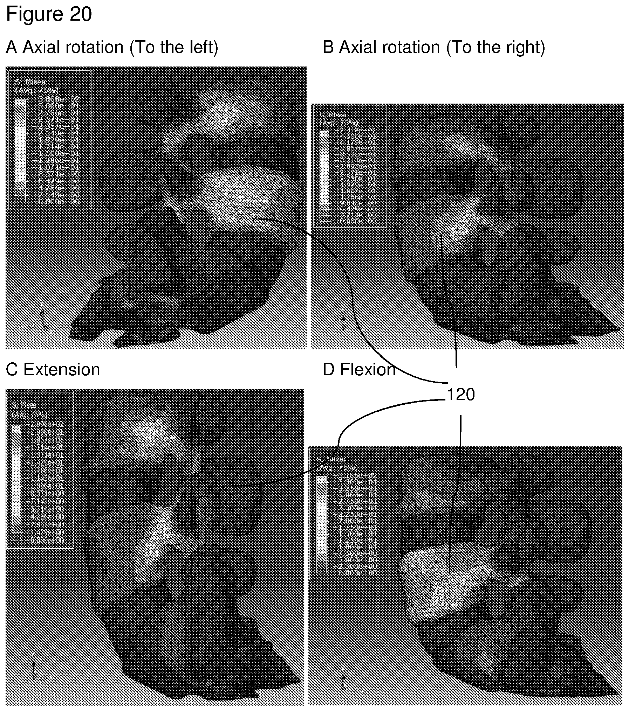

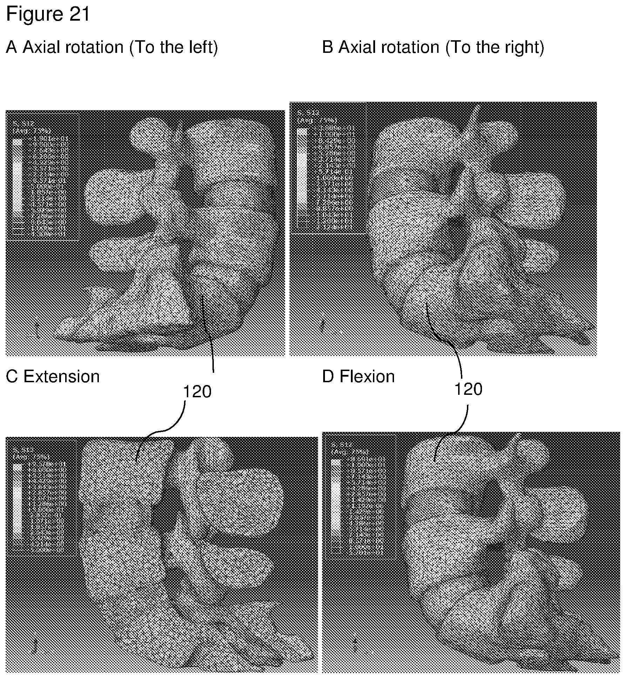

Laminar facture may lead to back pain and serious illness which can be explained in biomechanics behaviour. The modelled laminar fracture of L5 120 is again shown in Figure 20. Figure 20 shows the axial rotation (To the left) with relatively large stress concentration within the pedicle of L5. Similar results for axial rotation (To the right) with 45Mpa of stress occurs in the pedicle of L5 in the left (Figure 20(b)). There was little difference for extension compared with healthy lumbar spine (Figure 20(c)), but 15Mpa extra for flexion loading as shown in Figure 20(d). In addition, a laminar defect can increase the chance of fracture to the vertebrae in different regions. As shown in Figure 21(a), shear stress occurs by the right side of pedicle. For Figure 21(b) shear stress increased to 12Mpa. Both are the axial rotation loading conditions. No or little increment of shear stress for extension and flexion was shown (Figure 21 (c & d).

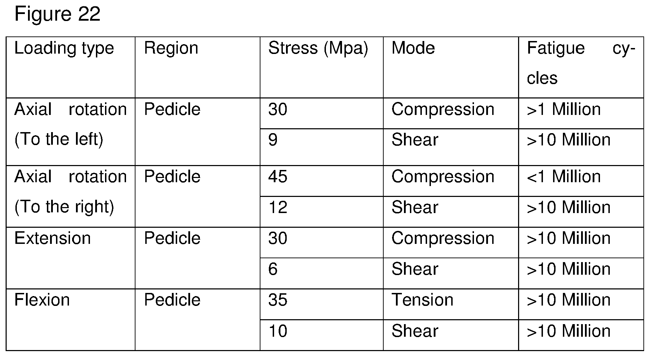

Figure 22 shows a table showing the overall result of number fatigue cycles prediction. For axial rotation (To the right), the compression stress is about 45Mpa and has reduced the number of fatigue cycles to less than 1 million cycles. This is because of the unconnected right laminar (fracture) which is unable to share the load from one side to another, therefore stress is concentrated on one side and no longer fully transmitting to S1 to equivalent the stress distribution. Undoubtedly, the discs are the major spinal components for the absorption of impact energy, particularly during the loading period. Through deformation of the discs, loads can be transmitted gently in order to prevent spinal injury. This suggests that with the laminar defect on right side of the L5 there is a risk of causing another fracture in the left side. Thus there is a risk of serious illness (spondylolysis or fatigue fracture) if no further treatment or implantation taken.

In another example of the software according to the invention the software further comprises inputs from an artificial intelligence (AI) program. The AI Program comes with a generic 3D patient model with anatomical landmarks. As described above a patient specific CT scan is performed and from that a three dimensional model is created. The AI program then personalises the generic 3D model with patient specific data. Then the AI program with a preprogrammed database is used to automatically recognise surface landmarks on the model. Then, anonomised patient specific landmarks augment and iteratively improve the generic model. The difference between generic and patient specific anatomical landmarks gives parameters for parametric automatic initial design proposition for the implant. A technician or surgeon will review the initial design and edit it within certain limits/parameters pre-verified by FE analysis on the generic model. The technician or surgeon is then allowed to manually change the design when required. Every time the technician or surgeon manually changes or modifies the design, the AI program will capture and store the manual changes into the database so that it can be called on for the next job or patient. In this way the software learns at the same time it improves the design for the next job.

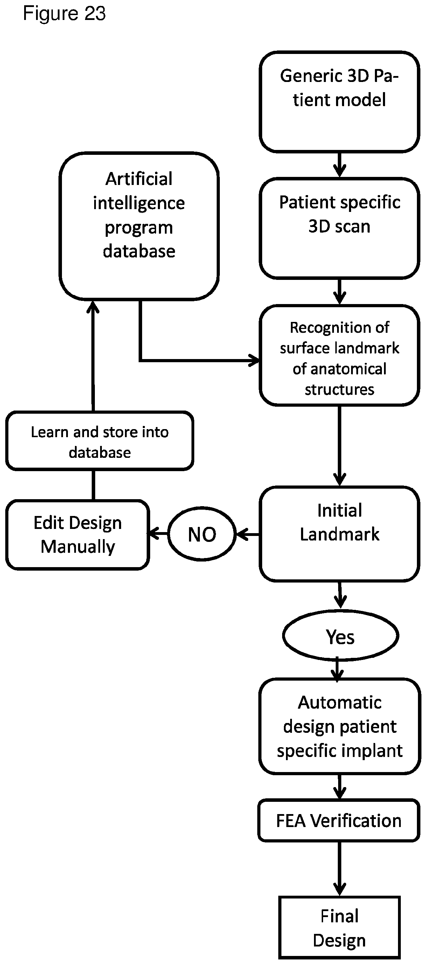

If the design is confirmed it will then be sent to FE analysis for verification within the boundary conditions. This includes the ability for remodelling, if the optimum design is not reached. Figure 23 shows a flow chart describing this exemplified automatic design and learning program work flow.

## Claims

1. An implant (1) for use in the body, the implant being **characterised in that**,
   the shape of at least one portion of the surface (32) of the implant (1) matches the surface shape of at least one portion of at least one body part; and
   the at least one portion of the surface (32) of the implant (1) is coated with a layer of adhesive.

2. An implant (1) according to claim 1, wherein the adhesive is curable by external input of a first energy, and the implant (1) comprises at least one conduit (4) whereby said first energy may be introduced into the adhesive, the at least one conduit (4) extending through the body of the implant (1) and opening at the at least one portion of the surface (32).

3. An implant according to claim 2, wherein the energy is electromagnetic radiation energy, ultrasound energy or thermal energy.

4. An implant according to claim 3, wherein the electromagnetic radiation energy is UV light derived from a UV light source.

5. An implant according to claim 4, wherein the adhesive cures within 90 seconds under external input of energy.

6. An implant according to any preceding claim, wherein the implant (1) comprises a flange (30) extending around the periphery of the implant (1) and protruding in a direction approximately at right angles away from the implant (1).

7. An implant according to any preceding claim, wherein the implant (1) comprises one or more protrusions positioned away from the periphery of the implant and protruding in a direction approximately at right angles away from the implant (1).

8. An implant according to any preceding claim, wherein the shape of the at least one portion of the surface (32) is formed in accordance with a set of data derived from the body part in three dimensions.

9. An implant according to claim 8, wherein the set of data is obtained by computer tomography, ultrasound imaging, cone beam computed tomography (CBCT) and/or magnetic resonance imaging.

10. An implant according to any preceding claim, wherein the material of the implant (1) is opaque.

11. An implant according to any preceding claim, wherein the adhesive is deactivated by exposure to a second external energy, wherein the first and second energies are different.

12. An implant according to any preceding claim, wherein the implant is designed by a software model configured to design the implant (1) for use in the body, the software model comprising;
scanning a patient body part,
generating a 3D model in silico of the patient body part from the scan,
generating a 3D model in silico of the implant wherein the implant model has an abutment surface that is a negative of the surface of the body part model,
predicting the loading pattern and/or fatigue stresses on the implant model using inputted patient data,
and regenerating the model of the implant in silico until the loading pattern and/or fatigue stresses are within a threshold value.

13. An implant according to claim 12, wherein the model provides data to validate the correct placement of the implant (1) in-silico prior to surgical placement.

14. An implant according to claim 12 or 13, wherein the model uses finite element analysis to predict the loading pattern on the body part.

15. An implant according to any of claims 12 to 14, further comprising inputting data from a database of manual iterations to further optimise the overall design of the implant (1).

**Patentansprüche**

1. Implantat (1) zur Verwendung im Körper, wobei das Implantat **dadurch gekennzeichnet ist, dass**
die Form von zumindest einem Abschnitt der Oberfläche (32) des Implantats (1) zu der Oberflächenform von zumindest einem Abschnitt von zumindest einem Körperteil passt; und
der zumindest eine Abschnitt der Oberfläche (32) des Implantats (1) mit einer Schicht aus Klebstoff beschichtet ist.

2. Implantat (1) nach Anspruch 1, wobei der Klebstoff durch Einwirkung einer ersten Energie von außen härtbar ist und das Implantat (1) mindestens eine Leitung (4) umfasst, durch welche die erste Energie in den Klebstoff eingeleitet werden kann, wobei die mindestens eine Leitung (4) sich durch den Körper des Implantats (1) erstreckt und an dem zumindest einen Abschnitt der Oberfläche (32) öffnet.

3. Implantat nach Anspruch 2, wobei die Energie elektromagnetische Strahlungsenergie, Ultraschallenergie oder Wärmeenergie ist.

4. Implantat nach Anspruch 3, wobei die elektromagnetische Strahlungsenergie UV-Licht ist, das von einer UV-Lichtquelle stammt.

5. Implantat nach Anspruch 4, wobei der Klebstoff innerhalb von 90 Sekunden unter äußerer Energieeinwirkung aushärtet.

6. Implantat nach einem der vorhergehenden Ansprüche, wobei das Implantat (1) einen Flansch (30) umfasst, der sich um den Umfang des Implantats (1) herum erstreckt und in einer Richtung ungefähr rechtwinklig vom Implantat (1) weg hervorsteht.

7. Implantat nach einem der vorhergehenden Ansprüche, wobei das Implantat (1) einen oder mehrere Vorsprünge umfasst, die vom Umfang des Implantats entfernt angeordnet sind und in einer Richtung ungefähr rechtwinklig vom Implantat weg hervorstehen.

8. Implantat nach einem der vorhergehenden Ansprüche, wobei die Form des mindestens einen Abschnitts der Oberfläche (32) in Übereinstimmung mit einem Datensatz gebildet wird, der von dem Körperteil in drei Dimensionen abgeleitet ist.

9. Implantat nach Anspruch 8, wobei der Datensatz durch Computertomographie, Ultraschallbildgebung, Cone Beam Computertomographie (CBCT) und / oder Magnetresonanzbildgebung erhalten wird.

10. Implantat nach einem der vorhergehenden Ansprüche, wobei das Material des Implantats (1) undurchsichtig ist.

11. Implantat nach einem der vorhergehenden Ansprüche, wobei der Klebstoff deaktiviert wird, indem er einer zweiten äußeren Energie ausgesetzt wird, wobei die erste und die zweite Energie unterschiedlich sind.

12. Implantat nach einem der vorhergehenden Ansprüche, wobei das Implantat durch ein Softwaremodell gestaltet ist, das dazu konfiguriert ist, das Implantat (1) zur Verwendung in dem Körper zu gestalten, wobei das Softwaremodell umfasst;
Scannen eines Körperteils eines Patienten,
Erzeugen eines 3-D-Modells in silico des Körperteils des Patienten aus dem Scan,
Erzeugen eines 3 D-Modells in silico des Implantats, wobei das Implantatmodell eine Anlageoberfläche aufweist, die ein Negativ der Oberfläche des Körperteilmodells ist,
Vorhersagen eines Belastungsmusters und/oder einer Ermüdungsbelastung an dem Implantatmodell unter Verwendung eingegebener Patientendaten,
und Umgestalten des Modells des Implantats in silico bis das Belastungsmuster und/oder die Ermüdungsbelastung unter einem Grenzwert liegen.

13. Implantat nach Anspruch 12, wobei das Modell Daten bereitstellt, um die korrekte Platzierung des Implantats in silico vor der chirurgischen Platzierung zu validieren.

14. Implantat nach Anspruch 12 oder 13, wobei das Modell eine Finite-Elemente-Analyse verwendet, um das Belastungsmuster für den Körperteil vorherzusagen.

15. Implantat nach einem der Ansprüche 12 bis 14, weiterhin umfassend das Eingeben von Daten aus einer Datenbank von manuellen Iterationen, um das Gesamtdesign des Implantats (1) weiter zu optimieren.

**Revendications**

1. Implant (1) destiné à être utilisé dans le corps, l'implant étant **caractérisé par le fait que** :

la forme d'au moins une partie de la surface (32) de l'implant (1) correspond à la forme de surface d'au moins une partie d'au moins une partie de corps ; et

l'au moins une partie de la surface (32) de l'implant (1) est revêtue d'une couche d'adhésif.

2. Implant (1) selon la revendication 1, dans lequel l'adhésif est durcissable par apport externe d'une première énergie, et l'implant (1) comprend au moins un conduit (4), ce par quoi ladite première énergie peut être introduite dans l'adhésif, l'au moins un conduit (4) s'étendant à travers le corps de l'implant (1) et s'ouvrant à l'au moins une partie de la surface (32).

3. Implant selon la revendication 2, dans lequel l'énergie est de l'énergie de rayonnement électromagnétique, de l'énergie ultrasonore ou de l'énergie thermique.

4. Implant selon la revendication 3, dans lequel l'énergie de rayonnement électromagnétique est de la lumière UV provenant d'une source de lumière UV.

5. Implant selon la revendication 4, dans lequel l'adhésif durcit en 90 secondes sous un apport externe d'énergie.

6. Implant selon l'une quelconque des revendications précédentes, dans lequel l'implant (1) comprend une bride (30) s'étendant autour de la périphérie de l'implant (1) et faisant saillie dans une direction approximativement à angle droit s'éloignant de l'implant (1).

7. Implant selon l'une quelconque des revendications précédentes, dans lequel l'implant (1) comprend une ou plusieurs saillies positionnées à distance de la périphérie de l'implant et faisant saillie dans une direction approximativement à angle droit s'éloignant de l'implant (1).

8. Implant selon l'une quelconque des revendications précédentes, dans lequel la forme de l'au moins une partie de la surface (32) est façonnée selon un ensemble de données déduites de la partie de corps en trois dimensions.

9. Implant selon la revendication 8, dans lequel l'ensemble de données est obtenu par tomodensitométrie, imagerie ultrasonore, tomographie volumétrique à faisceau conique (TVFC) et/ou imagerie par résonance magnétique.

10. Implant selon l'une quelconque des revendications précédentes, dans lequel le matériau de l'implant (1) est opaque.

11. Implant selon l'une quelconque des revendications précédentes, dans lequel l'adhésif est désactivé par exposition à une seconde énergie externe, les première et seconde énergies étant différentes.

12. Implant selon l'une quelconque des revendications précédentes, dans lequel l'implant est conçu par un modèle logiciel configuré pour concevoir l'implant (1) destiné à être utilisé dans le corps, le modèle logiciel comprenant :

numériser une partie de corps de patient,

générer un modèle 3D in silico de la partie de corps de patient à partir de la numérisation,

générer un modèle 3D in silico de l'implant, le modèle d'implant ayant une surface de butée qui est un négatif de la surface du modèle de partie de corps,

prédire le mode de chargement et/ou des contraintes de fatigue sur le modèle d'implant à l'aide de données de patient entrées,

et régénérer le modèle de l'implant in silico jusqu'à ce que le mode de chargement et/ou les contraintes de fatigue se situent dans les limites d'une valeur de seuil.

13. Implant selon la revendication 12, dans lequel le modèle fournit des données pour valider la mise en place correcte de l'implant (1) in silico avant une mise en place chirurgicale.

14. Implant selon la revendication 12 ou 13, dans lequel le modèle utilise une analyse par éléments finis pour prédire le mode de chargement sur la partie de corps.

15. Implant selon l'une quelconque des revendications 12 à 14, comprenant en outre l'entrée de données à partir d'une base de données d'itérations manuelles pour optimiser davantage la conception globale de l'implant (1).

*FIG. 1*

*FIG. 2*

FIG. 3

FIG. 4

**FIG. 5**

**FIG. 6**

FIG. 7

FIG. 8

## Figure 9

Figure 10

100

## Figure 11

| Element | Young's Modulus (Mpa) | Poisson's ratio | Reference |
|---|---|---|---|
| Cortical bone | 12000 | 0.3 | (*Lan C. C. et al., 2013; Peng X. Q. & Wang Y., 2011*) |
| Spongial bone | 100 | 0.3 | (*Peng X. Q. & Wang Y., 2011; Teo E. C. & Ng H. W., 2001*) |
| Annulus | 4 | 0.499 | (*Kurutz M., 2010; Fagan et al., 2002*) |
| Nucleus | 1 | 0.499 | (*Lan C. C. et al., 2013;Teo E. C. & Ng H. W., 2001*) |
| Facet joint | 6 | 0.299 | (*Jaumard N. V. et al., 2011; Byun D. H. et al., 2012*) |

Figure 12

Figure 13

| Load Scheme | | | |
|---|---|---|---|
| L4-S1 | Experiment | Literature(FE) | FE result |
| AR (clockwise) | 1.51 | 1.65 | 1.078 |
| AR (anticlockwise) | 1.4 | 1.54 | 1.14 |
| Extension | 3.43 | 3.41 | 3.2 |
| Flexion | 3.29 | 3.82 | 3.12 |

Figure 14

A Axial rotation (To the left)     B Axial rotation (To the right)

C Extension                100     D Flexion                100

Figure 15

| Mode | $S_{\theta}$ | $S_{r}$ |
|---|---|---|
| Tension | 97.8 | -7.46 |
| Compression | 149.8 | -17.96 |
| Shear | 36.6 | -2.9 |

Figure 16

A Axial rotation (To the left)          B Axial rotation (To the right)

C Extension          110          D Flexion          110

## Figure 17

A Axial rotation (To the left)          B Axial rotation (To the right)

C Extension          110          D Flexion          110

## Figure 18

| Loading type | Region | Stress (Mpa) | Mode | Fatigue cycles |
|---|---|---|---|---|
| Axial rotation (To the left) | Pedicle | 30 | Compression | >1 Million |
| | | 11 | Shear | >10 Million |
| | Laminar | 19.29 | Compression | >1 Million |
| | | 11 | Shear | >1 Million |
| Axial rotation (To the right) | Pedicle | 30 | Compression | >1 Million |
| | | 6 | Shear | >10 Million |
| | Laminar | 17.14 | Compression | >1 Million |
| | | 6 | Shear | >1 Million |
| Extension | Pedicle | 30 | Compression | >1 Million |
| | | 6 | Shear | >10 Million |
| | Laminar | 19.29 | Compression | >10 Million |
| | | 6 | Shear | >10 Million |
| Flexion | Pedicle | 30 | Tension | >10 Million |
| | | 7 | Shear | >10 Million |
| | Laminar | 19.29 | Tension | >10 Million |
| | | 7 | Shear | >10 Million |

Figure 19

120

# Figure 20

A Axial rotation (To the left)

B Axial rotation (To the right)

C Extension

D Flexion

120

# Figure 21

A Axial rotation (To the left)          B Axial rotation (To the right)

C Extension                              D Flexion

120                                      120

Figure 22

| Loading type | Region | Stress (Mpa) | Mode | Fatigue cycles |
|---|---|---|---|---|
| Axial rotation (To the left) | Pedicle | 30 | Compression | >1 Million |
| | | 9 | Shear | >10 Million |
| Axial rotation (To the right) | Pedicle | 45 | Compression | <1 Million |
| | | 12 | Shear | >10 Million |
| Extension | Pedicle | 30 | Compression | >10 Million |
| | | 6 | Shear | >10 Million |
| Flexion | Pedicle | 35 | Tension | >10 Million |
| | | 10 | Shear | >10 Million |

Figure 23

Flowchart:

- Generic 3D Patient model → Patient specific 3D scan → Recognition of surface landmark of anatomical structures → Initial Landmark
- Artificial intelligence program database → Recognition of surface landmark of anatomical structures
- Initial Landmark → NO → Edit Design Manually → Learn and store into database → Artificial intelligence program database
- Initial Landmark → Yes → Automatic design patient specific implant → FEA Verification → Final Design

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040053196 A

**Non-patent literature cited in the description**

- **ENDRES KIRA et al.** *Biomedical Engineering online,* 2008, vol. 7, 16